# EUROPEAN PATENT SPECIFICATION

(11) **EP 2 376 060 B1**
(45) Date of publication and mention of the grant of the patent: **19.09.2012**
(21) Application number: 09795398.8
(22) Date of filing: 14.12.2009
(51) Int. Cl.: A61K 9/10, A61K 9/51, A61K 9/00, A61K 47/34, G01N 1/00

(54) **Nanoparticle compositions**
Nanopartikelzusammensetzungen
Compositions de nanoparticules

(30) Priority: 15.12.2008 US 122464 P
(43) Date of publication of application: 19.10.2011
(73) Proprietor: Novartis AG, 4056 Basel (CH)
(72) Inventor: KISSEL, Thomas, 35032 Marburg (DE); PETERSEN, Holger, CH-4002 Basel (CH); RENETTE, Thomas, 35037 Marburg (DE); SEIDEL, Nina, CH-4002 Basel (CH)
(74) Representative: Bone, Alexander Marcus Thomas
(86) International application number: PCT/EP2009/067122
(87) International publication number: WO 2010/079052

(56) References cited:
- EP-A1- 1 249 232
- WO-A2-95/06077
- PINTO REIS, C. ET AL: "Nanoencapsulation I. Methods for preparation of drug-loaded polymeric nanoparticles." NANOMEDICINE, vol. 2, 2006, pages 8-21, XP002569395
- LAMBERT, O. ET AL: "Poly(ethylene carbonate) microspheres: manufacturing process and internal structure characterization." J CONTROL RELEASE., vol. 67, no. 1, 2000, pages 89-99, XP002608135

## Description

### FIELD OF THE INVENTION

The invention pertains to poly(ethylene carbonate) (PEC) nanoparticles comprising pharmacologically active agents, their production and their use as pharmaceutical compositions, in particular as nanoparticle suspensions.

### BACKGROUND OF THE INVENTION

Polymeric nanoparticles have been extensively studied as particulate carriers in the pharmaceutical and medical fields, as they provide promising advantages as drug delivery systems. Nanoparticles are generally defined as solid, submicron-sized drug carriers that may or may not be biodegradable. The term "nanoparticle" is a collective name for both nanospheres and nanocapsules. Nanospheres have a matrix type of structure. Drugs may be adsorbed at the sphere surface or encapsulated within the particles. Nanocapsules are vascular systems in which the drug is confined to cavity consisting of an inner liquid core surrounded by a polymeric membrane. In this case, the active substances are usually dissolved in the inner core but may also be adsorbed to the capsule surface. Nanoparticles are receiving considerable attention for the delivery of therapeutic drugs.

In particular, injectable nanoparticle carriers are believed to have the ability to revolutionize disease treatment due to their spatial and temporal controlled drug delivery mechanism. For example, spatially localizing the release of toxic and other potent drugs only at specific therapeutic sites can lower the overall systemic dose and damage that these drugs would otherwise produce. Temporarily controlling the release of a drug can also help to decrease unwanted side effects that might otherwise occur due to the natural circadian fluctuations of chemical levels throughout the body. The overall benefit of these improvements in disease treatment would be an increase in patient compliance and quality of life. Typical polymers that have been used to produce polymeric nanoparticles are for example poly(methylmethacrylate), poly(ethyl cyanoacrylate), polyacrylamide, polyurethanes, poly(lactic acid), polystyrene, poly(lactide-co glycoloid) (PLGA) or poly(epsilon-caprolactone).

In order for a drug delivery device to achieve the above described benefits, it must be present in the bloodstream long enough to reach or recognize its therapeutic side of action. However, the opsonization or removal of nanoparticulate drug carriers from the body by the mononuclear phagocytic system (MPS), also known as the reticulo-endothelial system (RES), is a major obstacle to the realization of these goals. The macrophages of the MPS have the ability to remove unprotected nanoparticles from the bloodstream within seconds of intravenous administration, rendering them ineffective as side specific drug delivery devices. These macrophages, which are typically Kupffer cells, or macrophages of the liver, cannot directly identify the nanoparticles themselves but rather recognize specific opsonin proteins bound to the surface of the particles.

The polymers presently used for producing nanoparticles have the disadvantage that they are either rapidly recognized and accordingly removed by the macrophage system or that they are not stable against hydrolysis. One widely used method to slow down the opsonization of nanoparticles (and accordingly the elimination by macrophages) is the use of surface adsorbed or grafted shielded groups which can block the electrostatic and hydrophobic interactions that help opsonins bind to particle surfaces. These groups tend to be long hydrophilic polymer chains and non-ionic surfactants. Some examples of polymer systems that have been tried as shielding groups in order to slow down opsonization and accordingly macrophage elimination include polysaccharides, polyacrylamide, polyvinyl alcohol, PEG and PEG containing copolymers. Relevant prior art includes EP 1249 252 and Pinto Reis, C. et Al,: "Nano Encapsulation I. Methods for Preparation of Drug Loaded Polymeric Nanoparticle", nanomedicing Nul.2, 2006, PAGES 8-21.

It is the object of the present invention to provide nanoparticles and in particular nanoparticle suspensions having improved stability properties that are suitable as pharmaceutical compositions.

### BRIEF SUMMARY OF THE INVENTION

According to one aspect, the application pertains to a pharmaceutical composition comprising poly(ethylene carbonate) (PEC) nanoparticles comprising a pharmacologically active agent. These PEC nanoparticles not only depict a physical and chemical stability, but furthermore a biological stability against phagocytosis compared to conventional polymeric nanoparticles. Due to this lower phagocytosis rate, the PEC nanoparticles are less quickly eliminated by the macrophage system. These important characteristics make the PEC nanoparticles of the present application suitable for use as a pharmaceutical composition for the delivery of pharmacologically active agents. Its unique properties make PEC a suitable nanoparticle carrier for the controlled or sustained release of the comprised, preferably encapsulated, pharmacologically active agent. In particular, the improved stability against phagocytosis allows the use of the PEC nanoparticles according to the present invention as a parenteral depot for releasing an encapsulated pharmacologically active agent upon biodegradation of the PEC nanoparticle, thereby making said pharmacologically active agent systemically available over a longer release period. Therefore, the use of PEC nanoparticles as carriers for pharmacologically active agents have significant advantages over polymeric nanoparticles known in the prior art.

According to a further aspect the application pertains to a suspension of poly(ethylene carbonate) nanoparticles, preferably incorporating a pharmacologically active agent. A respective PEC nanoparticle suspension is physically stable and is suitable for subcutaneous local delivery. According to a further aspect the application pertains to the use of a suspension of PEC nanoparticles for preparing a pharmaceutical composition.

According to a further aspect, the application pertains to a method of manufacturing a pharmaceutical composition comprising poly(ethylene carbonate) nanoparticles comprising a pharmacologically active agent, in particular by preparing a PEC nanoparticle suspension by the solvent displacement method.

Other objects, features, advantages and aspects of the present application will become apparent to those skilled in the art from the following description and appended claims.

### BRIEF DESCRIPTION OF THE FIGURES

**Figure 1****:** shows the regulation of the particle size of the unloaded nanoparticles by variation of the polymer concentration (Figure 1 a for PEC 95 and Figure 1b for PEC 99). The figure shows the particle size in nanometers obtained by PCS for three different batches using different polymer concentrations (see example 4). The average PDI of the triplicates is indicated in parentheses after the charge name. The standard deviation of the sizes is indicated by the bars.
**Figure 2****:** shows the regulation of the particle size of the loaded nanoparticles by variation of the polymer concentration. The figure shows the particle size in nanometers obtained by PCS for three different batches using different polymer concentrations. The standard deviation of the sizes is indicated by the bars.
**Figure 3****:** shows the change of the size of PEC 95 nanoparticles at different temperatures. The drawn-through line corresponds to the average size of the triplicates prepared at 4°C, which runs through all temperature changes of a climate incubator (see example 5.2).
**Figure 4****:** shows the change of the size of PEC 99 nanoparticles at different temperatures. The drawn-through line corresponds to the average size of the triplicates prepared at 4°C, which runs through all temperature changes of a climate incubator (see example 5.2).
**Figure 5****:** shows the results of a swelling test of nanoparticles composed of PEC 99 (A) or PEC 95 (B) analysed by PCS (see example 5.3). The change of the size is indicated during the evaporation process. Nanoparticles are prepared using PEC 99 or PEC 95. After injection into PVA solution (T=0) aliquots are taken for the time points depicted and analyzed for particle size using PCS. Only the data for the first 24 hours (t=0. 30 min, 60 min, 90 min, 120 min, 3h, 4h, 5h, 6h, 7h and 24h) are shown. The error bar indicates the standard deviation of the triplicates.
**Figure 6a****:** shows the analysis of PEC 95 particles (0.1 mg / 5 ml) using atomic force microscopy (AFM). **Figure 6b** shows the analysis of PEC 95 particles (3 mg 5ml) using atomic force microscopy (AFM).
**Figure 7****:** shows- the analysis of PEC 99 particles (0.1 mg / 5 ml) using atomic force microscopy (AFM).
**Figure 8****:** shows the swelling characteristics of PEC 95 (3 mg / ml) within the first half hour of the evaporation process during preparation of the particles (see example 5.3).
**Figure 9****:** shows the fluorescence as determined via FACS for loaded PEC 95 and PEC 99 nanoparticle suspensions at 37°C (see example 8).
**Figure 10****:** shows the fluorescence as determined via FACS at 4°C and 37°C (see example 8) for different nanoparticles plus NaN₃.

### DETAILED DESCRIPTION OF THE INVENTION

According to one aspect of this application, a pharmaceutical composition is provided comprising poly(ethylene carbonate) nanoparticles comprising a pharmacologically active agent.

The use of poly(ethylene carbonate) (PEC) as a matrix material for a polymeric nanoparticle is advantageous over the use of common biodegradable and hydrolytically degradable polymers, such as PLGA or poly(epsilon-caprolactone). PEC nanoparticles have a high loading capacity and PEC is chemically stable in aqueous solutions and degrades only *in vivo.* PEC is biodegradable *in vivo* and *in vitro* in particular by superoxide radical anions O₂⁻¹, which said anions are predominantly produced *in vivo* by inflammatory cells. This non-hydrolytic biodegradation by cells producing O₂⁻¹ is rather unique among the biodegradable polymers. Furthermore, their biodegradation products of PEC only have a very low or even no toxicity.

Furthermore, it was surprisingly found by the inventors that PEC nanoparticles are much less phagocytised and accordingly removed/eliminated by macrophages than other polymeric nanoparticles known in the prior art, such as for example polystyrene nanoparticles. Due to this important characteristic, the PEC nanoparticles of the present invention are biologically more stable in the sense that the PEC nanoparticles are much less attacked and accordingly eliminated by macrophages and accordingly release the contained drug over a longer time period than conventional polymeric nanoparticles.

These important aspects, the physical stability of the PEC nanoparticles, the chemical stability of the PEC nanoparticles and their biological stability against phagocytosis, make the PEC nanoparticles of the present invention suitable for use as a pharmaceutical composition for the delivery of pharmacologically active agents. Its unique properties make PEC a suitable nanoparticle carrier for the controlled or sustained release of the comprised, preferably encapsulated, pharmacologically active agent. As used herein, the term "sustained release" or "controlled release" means that the PEC nanoparticle used releases no more than 10, 20, 30, 40 or 50% to 60, 70, 80, or 90% by weight of the pharmacologically active agent dissolved or dispersed therein within 3 to 10, e.g. 7, days after implantation of, the device into a human or animal body.

In particular, the improved stability against phagocytosis allows the use of the PEC nanoparticles according to the present invention as a parenteral depot for releasing an encapsulated pharmacologically active agent upon biodegradation of the PEC nanoparticle, thereby making said pharmacologically active agent systemically available over a longer release period. Therefore, the use of PEC nanoparticles as carriers for pharmacologically active agents have significant advantages over polymeric nanoparticles known in the prior art.

In the present invention, the pharmaceutical composition comprises at least one pharmacologically active agent comprised within the PEC nanoparticles, and can be e.g. encapsulated, dissolved or dispersed therein. The nanoparticles of the present invention can be used for the controlled or sustained delivery of the pharmacologically active agent to the patient. The terms "sustained release" or "controlled release" as used herein shall be used as defined above.

The PEC nanoparticles of the present invention have a diameter ranging from 1 - 1000 nm. The diameter of the nanoparticles may be less than 800, 750, 700, 650, 600, 550, 500 nm and/or less than 450 nm, preferably with a narrow size distribution. Nanoparticles of a smaller diameter enable the use of smaller needle sizes in case a nanoparticle suspension is used that is supposed to be injected.

As used herein, the term "pharmacologically active agent" comprises any substances which may yield a physiological response when administered to a living organism. Such substance is usually administered in a therapeutically effective amount. As used herein, the term "therapeutically effective amount" generally refers to an amount or concentration which is effective in reducing, eliminating, treating, preventing or controlling the symptoms or development of a disease or condition affecting a mammal. Controlling is intended to refer to all processes wherein there may be a slowing, interrupting, arresting or stopping of the progression or development of a disease and conditions affecting the mammal. However, "controlling" does not necessarily indicate a total elimination of all disease and condition symptoms, and is intended to also include prophylactic treatment. The appropriate therapeutically effective amount is known to one of ordinary skill in the art as the amount varies with the therapeutic compound being used and the indication which is being addressed. As used herein the meaning of the terms "pharmaceutical active agent", "active ingredient", "pharmacologically active compound", "active substance" or "drug substance" is to be understood as equivalent.

Many different pharmacologically active agents can be delivered/formulated with the PEC nanoparticles according to the present invention. Examples of therapeutic classes of drugs include, but are not limited to anti-hypertensives, anti-anxiety agents, anti-clotting agents, anti-convulsants, blood glucose-lowering agents, anti-histamines, anti-tussives, anti-neoplastics, beta-blockers, anti-inflammatory agents, anti-psychotic agents, cognitive enhancers, anti-atherosclerotic agents, cholesterol reducing agents, anti-obesity agents, autoimmune disorder agents, anti-impotence agents, anti-bacterial and anti-fungal agents, immunosuppressant agents, hypnotic agents, anti-depressants, antiviral agents, antibiotics, chemotherapeutic agents, contraceptives, sedatives, steroids, vitamins, enzymes, antigens and combinations of the foregoing.

The PEC nanoparticles are particularly suitable for pharmacologically active agents, which are pharmacologically active in low amounts and need to have an uninterrupted blood level during extended periods, such as e.g. hormones, peptides or proteins, chemical entities with high affinity to biological targets, e.g. somatostatins, biphosphonates, interferon, and interleukins. The PEC nanoparticle suspensions according to the present invention are in particular suitable for the delivery of pharmacologically active agents that are unstable and will disintegrate after oral use or in the gastro-intesitinal system and thus preferably are administered parenterally.

The strength of the PEC polymer is its encapsulation of proteins without denaturizing them. Upon in vivo degradation of the PEC, no acidic microenvironment is generated (which is typically observed for PLGA polymers) which is another advantage as also acid-labile proteins can be applied in an PEC depot.

The pharmacologically active agent used in the present invention may be selected from the group consisting of chemical compounds, biologically active agents, nucleic acids, peptides and proteins. The term "biologically active agent" as used herein refers to a agent having the potential to react with biological components. More particularly, biologically active agents utilized in this specification are designed to change the natural processes associated with a living cell. For purposes of this specification, a cellular natural process is a process that is associated with a cell before delivery of a biologically active agent. Examples of biologically active agents include, but not limited to, pharmaceuticals, proteins, peptides, polypeptides, enzyme inhibitors, hormones, cytokines, antigens, viruses, oligonucleotides enzymes and polynucleotides are examples of biologically active agents.

The term "protein" as used herein refers to a polypeptide (i.e., a string of at least two amino acids linked to one another by peptide bonds). Proteins may include moieties other than amino acids (e.g., may be glycoproteins, proteoglycans, etc.) and/or may be otherwise processed or modified. Those of ordinary skill in the art will appreciate that a "protein" can be a complete polypeptide chain as produced by a cell (with or without a signal sequence), or can be a characteristic portion thereof. Those of ordinary skill will appreciate that a protein can sometimes include more than one polypeptide chain, for example linked by one or more disulfide bonds or associated by other means. Useful modifications include, e.g., terminal acetylation, amidation, etc. In some embodiments, proteins may comprise natural amino acids, non-natural amino acids, synthetic amino acids, and combinations thereof. The term "peptide" is generally used to refer to a polypeptide having a length of less than about 100 amino acids. Examples of proteins and peptides include, but are not limited to, cytokines, e.g., interleukins, G-CSF, M-CSF, GM-CSF or LIF, interferon, erythropoetins, cyclosporins, or hormones, or their analoges.

The PEC nanoparticles are particularly suitable as carriers for pharmacologically active agent having a low molecular weight (MW) of less than 2500 Da. Furthermore, according to a preferred embodiment, the pharmacologically active agent has a lipophilic character or comprises a lipophilic group. Respective agents are efficiently encapsulated by the PEC matrix to form the PEC nanoparticles of the present invention.

The pharmacologically active agent may be selected from the group consisting of the class of somatostatin analogues, e.g. pasireotide, lanreotide, octreotide, vapreotide and salts thereof, biphosphonates, e.g. zoledronic acid, and salts thereof, and lipid altering drugs. A somatostatin analogue is a compound having a somatostatin-like activity. Several biological active oligopeptide are known which have somatostatin-like activity which inhibit the growth hormone release from mammalian pituitary gland. These biologically active oligopeptides are well known in the art together with the mechanism of action thereof, which is disclosed for instance by Weckbecker et al. 2003 (Nature Reviews, Vol. 2, p. 999-1016) and Murray et al. 2004 (J Clin Invest, Vol. 114 p. 349-356), which are both herein incorporated by reference. Apart from somatostatin itself, these biologically active oligopeptides include but are not limited to octreotide, lanreotide, vapreotide and pasireotide, together with the pharmaceutically acceptable salts thereof, preferably the acetate.

For the purposes of the present invention, the term "bisphosphonates" refers to a drug containing two phosphonate groups. Biphosphonates are classified into N-containing biphosphonates like pamidronate, neridronate, alendronate, ibandronate, or risedronate and Non-N containing biphosphonates like etidronate, clodronate and tiludronate. For example, zoledronic acid can used for the drug delivery system of the invention. The bisiphosphonate zoledronic acid is designated chemically as (1-Hydroxy-2-imidazol-1-yl-phosphonoethyl) phosphonic acid monohydrate and its structural formula is set forth as Formula A:

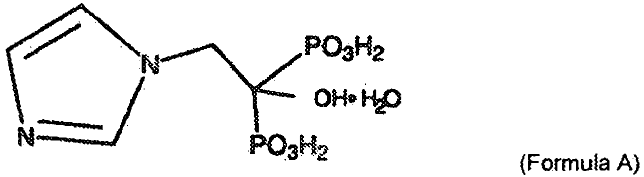

Alternatively, also lipid altering drugs may be loaded in the nanoparticles of the present invention. For the purposes of the present invention, the expression "lipid altering drugs" refers to any drug that changes the blood concentration of lipids or lipoproteins like e.g. cholesterol, triglycerides, the very low density lipoprotein (VLDL), the low density lipoprotein (LDL), the intermediate density lipoprotein (IDL), the high density lipopoprotein (HDL), the very high density lipopoprotein (VHDL), the lipopoprotein a and the chylomikrones. These drugs include but are not restricted to cholesterol absorption inhibitors, niacin, fibrates or statins. Non-limiting examples for lipid altering drugs are Lescol, niacin receptor activators and activators of the thyroid receptor beta.

Alternatively, also immunosuppressant agents may be loaded in the nanoparticles of the present invention. Non-limiting examples for immunosuppressant agents are cortisol, dexamethasone, alkylating agents, nitrogen mustards, nitrosoureas, and azathioprine, rapamycin, cyclosporine, FK506, and methotrexate.

The pharmacologically active agent may be present in an amount up to about 70% by weight of the composition, from about 0.5% to about 60% by weight of the composition, from about 10% to about 40% by weight of the composition, or from about 1.0 to 10% by weight of the composition. It is intended, however, that the choice of a particular level of pharmacologically active agent will be made in accordance with factors well-known in the pharmaceutical arts, including the solubility of the pharmacologically active agent in the PEC used, mode of administration and the size and condition of the subject.

Examples of therapeutic classes of drugs include, but are not limited to, anti-hypertensives, anti-anxiety agents, anti-clotting agents, anti-convulsants, blood glucose-lowering agents, decongestants, anti-histamines, anti-tussives, anti-neoplastics, beta-blockers, anti-inflammatory agents, anti-psychotic agents, cognitive enhancers, anti-atherosclerotic agents, cholesterol reducing agents, anti-obesity agents, autoimmune disorder agents, anti-impotence agents, anti-bacterial and anti-fungal agents, hypnotic agents, antibiotics, anti-depressants, antiviral agents and combinations of the foregoing.

The PEC polymer(s) used in the preparation of the PEC nanoparticles of according to the present invention may comprise ethylene carbonate units of the formula A:

-(-C(O)-O-CH₂-CH₂-O-)- (Formula A)

having at least one of the following characteristics:
- it has an ethylene carbonate content of 70 to 100 Mol %, and/or
- it has an intrinsic viscosity of 0.4 to 4:0 dl/g as measured in chloroform at 20° C, and/or
- it has a glass transition temperature of from 5 to 50° C.

The PEC polymer used has an ethylene carbonate content from 70 to 100 Mol %. Preferably, the ethylene content of the PEC polymer is from 80 to 100%, preferably from 90 to 99.9 %.

The PEC polymer used has an intrinsic viscosity from 0.4 to 4.0 dl/g measured in chloroform at 20°C. Preferably, the PEC polymer has an inherent viscosity, measured at 20°C and a concentration of 1 g/dl in chloroform of 0.4 to 3.0 dl/g.

The PEC polymer used has a glass transition temperature from 5°C to 50°C, preferably from 15° to 25°C.

The PEC polymer used has a molecular weight of less about 2000 kDa. Preferably, the molecular weight is less than 500 kDa, as can be determined e.g. by gel permeation chromatography with methylene chloride as the eluent and polystyrol as reference.

In a further embodiment, the PEC polymer may be present in form of a (co)-polymer containing e.g. as a co-unit the ethylene oxide unit of the formula B:

-(-CH₂-CH₂-O-)- (Formula B).

The PEC polymer, if containing ethylene oxide units, has a random distribution of ethylene carbonate and ethylene oxide units according to the sum formula A*ₘ* - B*ₙ* =

- (C(O)-O-CH₂-CH₂-O-)-ₘ-(-CH₂-CH₂-O-)-ₙ (Formula C)

in which *ml* (*n*+*m*) x 100 = 70 to 100. However, most of the ethylene oxide units in the PEC polymers of the present invention have, statistically, adjacent ethylene carbonate units, especially in those cases in which the molar ratio of ethylene oxide units is small. That means that in these cases most of the resulting ether functions are distributed randomly between carbonate functions along the polymer chain. One of ordinary skill would understand that ¹H-NMR spectra of the products of the invention in DCCl₃ confirm this assumption

The PEC polymer of the present invention are stable for several hours in hot water (90-100°C) without considerable molecular weight reduction. A significant increase of the glass transition temperature is observed after exposure to boiling bidistilled water during hours, e.g., up to above 18°C, e.g., 28°C.

The PEC polymer may be present in an amount up to about 99% by weight of the composition, from about 0.5% to about 80% by weight of the composition, from about 10% to about 30% by weight of the composition, or from about 1 to 10% by weight of the composition.

According to one embodiment, the pharmaceutical composition takes the form of a nanoparticle suspension. The use of a biodegradable PEC nanoparticle suspension is advantageous over the use of PEC microparticles, because nanoparticle suspensions in contrast to microparticle suspensions do not sediment but form physically stable suspensions.

Several pharmacokinetic profiles can result following the injection of nanosuspensions. Depot delivery via subcutaneous, intramuscular or intradermal routes offers prolonged drug release, because of the ability to load more drug amounts safely into a small injectable volume. The great loading capacity of the PEC nanoparticle suspension is an important advantageous feature of the nanoparticle suspension of the present invention.

Accordingly, the PEC nanoparticles of the present invention can be advantageously used in form of a suspension, in particular in form of a parenteral formulation. As used herein the term "parenteral formulation" denotes to a composition that is given by routes other than the digestive tract using a syringe and a needle or catheter. This includes intravenous, intraarterial, intramuscular, intracardial, subcutaneous, intraosseus, intradermal, intrathecal, intraperitoneal, intravesical, transdermal, transmucosal, epidural and intravitreal application. The PEC nanoparticle suspensions are in particular suitable for subcutaneous local delivery.

Due to their beneficial stability profile, the PEC nanoparticles of the present invention can be used in form of a ready to use parenteral depot, which can be injected e.g. subcutaneous. For this purpose, also a very small needle size (for example 27 G or less) can be used due to the small size of the PEC nanoparticles. These characteristics make the respective depot formulation also feasible for home use and self-administration.

Several parenteral application forms and respective drug formulations are known by the person skilled in the art and include injection, infusion, concentrate and implantate. The pharmaceutical composition comprising PEC nanoparticles may be made by working them up with suitable galenic excipients and optionally bringing them in appropriate dispensers.

In order to increase the stability of the PEC nanoparticle suspensions, the formulation may further comprise stabilizers, such as for example surfactants which prevent the agglomeration or precipitation of the nanoparticles. For example, ionic or non-ionic surfactants can be used. Examples of surfactants include, but are not limited to, fatty acids; alkyl sulfonates; polyoxyethylene fatty acids; sorbitan derivatives; polyoxyethylene sorbitan fatty acid esters; lecithin; phospholipids; mono-, di- and triglycerides; and mixtures thereof. The use of a surfactant in pharmaceutical compositions is well-known to the skilled person. For convenience reference is made to Remington: The Science and Practice of Pharmacy. 20th edition, 2000.

In a further aspect of the application, the pharmaceutical composition comprising the PEC polymer and the pharmacologically active agent may further comprise pharmaceutically acceptable excipients, e.g. ionic or non-ionic surfactants, binding agents or adhesives, antioxidants, lubricants and/or pH modifiers. It will be appreciated that such further ingredients are well known in the art. Hence, the PEC nanoparticles may comprise further polymers and/or additives. Example include radical scavengers in or on the nanoparticle, such as for example maenadione and/or vitamin C. The respective additives can be embedded in the (co)-polymer and may decrease e.g. the degradation rate of the poly(ethylene carbonate) thereby allowing a further prolongation of the drug release and accordingly the depot activity of the nanoparticle formulation.

Examples of such surfactants include, but are not limited to, reaction products of a natural or hydrogenated castor oil and ethylene oxide, such as the CREMOPHOR series from BASF Corp. (Mt. Olive, NJ); polyoxyethylene fatty acid esters that include polyoxyethylene stearic acid esters, such as the MYRJ series from Uniqema (New Castle, DE); sorbitan derivatives, such as the TWEEN series from Uniqema (New Castle, DE); polyoxyethylene-polyoxypropylene co-polymers and block co-polymers or poloxamers, e.g., SYNPERONIC PE/F 87/108/127L44 from Uniqema and PLURONIC (Lutrol F127) from BASF; polyoxyethylene alkyl ethers; water-soluble tocopheryl PEG succinic acid esters available from Eastman Chemical Co. (Kingsport, TN) with a melting point of about 36°C; PEG sterol ethers having, e.g., from 5-35 [CH2-CH2-O] units, e.g., 20-30 units, e.g., SOLULAN C24 (Choleth-24 and Cetheth-24) from Chemron (Paso Robles, CA); polyglycerol fatty acid esters, such as DECAGLYN, HEXAGLYN and TETRAGLYN from Nikko Chemicals (Tokyo, Japan); and alkylene polyol ether or ester compounds.

Examples of binding agents or adhesives include, but are not limited to, either individually or in combination, acacia; tragacanth; sucrose; gelatin; glucose, starches such as, but not limited to, pregelatinized starches; alginic acid and salts of alginic acid; magnesium aluminum silicate; PEG; guar gum; polysaccharide acids; bentonites; povidone, for example povidone K-15, K-30 and K-29/32; polymethacrylates: HPMC; hydroxypropylcellulose; and ethylcellulose.

Examples of antioxidants include, but are not limited to, ascorbic acid and its derivatives, tocopherol and its derivatives, butyl hydroxyl anisole and butyl hydroxyl toluene: Vitamin E as α-tocopherol is particularly useful.

Examples of lubricants include, but are not limited to, magnesium stearate, calcium stearate, sucrose fatty acid ester, polyethylene glycol, talc and stearic acid.

Examples of pH modifiers include, but are not limited to, include, but are not limited to NaOH, LiOH, KOH, Na₂CO₃, NaHCO₃, K₂C_{O3}, KHCO₃, NaH₂PO₄, Na₂HPO₄, Na₃PO₄, KH₂PO₄, K₂HPO₄, K3PO₄, megluamine, Ca(OH)₂, Mg(OH)₂, Zn(OH)₂, Al(OH)₃-, pyridoxine, triethanolamine, ammonium hydroxide, cytosine, diethylamine, meglumine, ornithine, glycine, lysine, arginine, valine, proline, aspartic acid, alanine, asparagine, isoleucine, leucine, methionine, threonine, choline hydroxide, procaine, diethylethanolamine, glucosamine, guanine, nicotinamide, piperazine, guanidine, olamine, piperidine, triethylamine, tromethamine, benzathine, benzathine, adenine, mixtures thereof and the like.

In a further aspect of the application, the poly(ethylene carbonate) nanoparticles are in the form of a suspension. The characteristics of the PEC nanoparticle in the suspension are already described above, we refer to the above disclosure.

According to one embodiment, the PEC nanoparticle suspension comprises at least one pharmacologically active agent and has at least one of the following additional characteristics:
a) the PEC polymer has at least one of the following characteristics:
   - it has an ethylene carbonate content of 70 to 100 Mol %,
   - it has an intrinsic viscosity of 0.4 to 4.0 dl/g as measured in chloroform at 20° C, and/or
   - it has a glass transition temperature of from 5 to 50° C;
b) the PEC nanoparticle suspension is a parenteral formulation;
c) the PEC nanoparticle suspension has sustained release characteristics;
d) the PEC nanoparticle suspension is a parenteral depot formulation;
e) the diameter of the nanoparticles of the suspension is less than 1000 nm, 800 nm, 750 nm, 700 nm, 650 nm, 600 nm, 550 nm, 500 nm and/or less than 450 nm; and/or
f) the PEC polymer used has a molecular weight of less 2000 kDa.
The PEC nanoparticles of the suspension comprise a pharmacologically active agent, in particular a pharmacologically active agent as described above. The PEC nanoparticles of the suspension optionally may include additional pharmaceutically acceptable excipients, e.g. ionic or non-ionic surfactants, adhesives, stabilizers, antioxidants, lubricants and/or pH modifiers and/or additives, e.g. radical scavengers in or on the nanoparticles, such as for example maenadione and/or vitamin C. We refer to the above detailed disclosure which also applies here.

The present invention also pertains to the use of a respective PEC nanoparticle suspension for preparing a pharmaceutical composition.

The invention also provides a method for manufacturing a pharmaceutical composition as described above by preparing a suspension of poly(ethylene carbonate) nanoparticles comprising a pharmacologically active agent.

Nanoparticle suspensions can be obtained by various techniques. Suitable methods for preparing a nanoparticle suspension are described in detail for example in Rice et al., Nanomedicine: Nanotechnology, Biology, and Medicine 2 (2006) 8-21, herein fully incorporated by reference which can also be used for preparing the PEC nanoparticle suspensions described herein. In particular, the solvent evaporation method, the salting out and the solvent displacement method are most advantageous. The solvent displacement method was used subsequently as it renders nanoparticles of a uniform distribution size.

When using the solvent displacement method, the polymer is first dissolved in an organic solvent (for example acetone, acetonitrile, dimethyl sulfoxide (DMSO), N-1-methyl-2-pyrrolidone (NMP), chloroform, 1,4-dioxan, dimethylformamide (DMF), or N-2-pyrrolidone) which is miscible with water in a defined concentration. This mixture is then injected via a syringe or injection pump into an aqueous solution (for example, PBS buffer (pH 7.4, 0.1M) which can optionally comprise a stabilizer. Immediately, a rapid diffusion of the organic phase in the aqueous phase occurs, thereby leading to the creation of a colloidal, tyndallised system. This solution is mixed with a magnetic stirrer under normal pressure or reduced pressure until the organic solvent is completely evaporated. The size of the nanoparticles that can be produced by the solvent displacement method can be varied by changing different factors. Important factors that may influence the size is the polymer concentration, the concentration of the optional stabilizer in the aqueous solution, and the proportion of the organic to the aqueous phase, the diameter of the injection capillary, the injection velocity of the polymer/solvent mixture and also the temperature. In particular the polymer concentration is decisive. The solvent displacement method shows in particular for lipophilic compounds a high encapsulation efficiency and is accordingly very suitable in order to encapsulate lipophilic drug compounds, as is shown in the examples by the lipophilic fluorescent marker coumarin-6 as a model substance.

Examples of organic solvents include, but are not limited to, acetone, acetonitrile, dimethyl sulfoxide (DMSO), N-1-methyl-2-pyrrolidone (NMP), chloroform, 1,4-dioxan, dimethylformamide (DMF), or N-2-pyrrolidone; preferably dimethyl sulfoxide (DMSO) or N-1-methyl-2-pyrrolidone (NMP).

For loading the nanoparticles with at least one pharmacologically active agent, a first stock solutions of the pharmacologically active agent is prepared by dissolving the pharmacologically active agent in organic solvent (for example acetone or acetonitrile) which is miscible with water in a defined concentration. The concentration of the pharmacologically active agent in the first stock solution may range from 1 to 500 micrograms per millilitre of organic solvent, preferably from 5 to 100 micrograms per millilitre of organic solvent, most preferably 25 to 7 micrograms per millilitre of organic solvent. A second stock solution is prepared by dissolving the PEC polymer in organic solvent (for example acetone or acetonitrile) which is miscible with water in a defined concentration. The concentration of the PEC polymer in the second stock solution may range from 0.1 to 100 milligrams per millilitre of organic solvent, preferably from 0.5 to 50 milligrams per millilitre, most preferably from 1 to 25 milligrams per millilitre. The necessary amounts of the first stock solution and the second stock solution are transferred with a pipette into an Eppendorf cup and filled with organic solvent to 1.5 ml. After vortexing and injection into the PVA solution, the desired end concentrations are obtained. Methods to determine the proper concentration and amount of stock solutions to obtain the desired end concentration are well-known to the skilled person.

Depending on the physical and chemical properties of the pharmacologically active agent, the drug loading content can vary between 0.1 and 70 % of the weight. When preparing the PEC nanoparticles comprising a pharmacologically active agent, the amount of PEC polymer compared to the pharmacologically active agent by weight can vary between 1 and 90 %, preferably approximately 30%.

According to one embodiment, the PEC polymers are dissolved in acetonitrile, as acetonitrile is miscible with water and dissolves the PEC polymer:

The solvent that is used to dissolve the PEC polymer is displaced by a second solvent, preferably containing an emulsifier. Details are also described in the example section. As emulsifier, polyvinyl alcohol, preferably Mowiol 18/88 can be used.

According to one embodiment, the size of the particles is regulated by the polymer concentration. It was shown by experiments, that a PEC polymer concentration of 0.1 mg / 5 ml PVA is suitable to obtain nanoparticles having a size of less than 200 nm. Concentrations of 3 mg / 5 ml PVA rendered nanoparticles having a size barely above 200 nm and accordingly are still in a very suitable range.

### EXAMPLES

### EXAMPLE 1: Experimental procedure for the synthesis of (poly ethylene) carbonate

PEC can be obtained from the reaction of CO₂ with ethylene oxide and subsequent polymerisation (see for example Acemoglu et al, Poly(ethylene carbonate)s part I: Syntheses and structural effects on biodegradation, Journal of controlled release, 1997, 49 (2,3): p. 263 - 275 and Vogdanis et al Carbon dioxide as a monomer, The polymerization of ethylene carbonate. Makromol. Chem. 1986. Rapid Commun. 7: p: 543-547, herein incorporated by reference).

The PEC polymers PEC 95 and PEC 99 that are subsequently used for the preparation of the nanoparticles exhibit the physical properties shown in Table 1. PEC 95 has an ethylene carbonate content [Mol%] of 97%.

**Table 1: Physical properties of poly(ethylene carbonate)s**

| | **PEC 95** | **PEC 99** |
|---|---|---|
| Molecular weight [kDa] | 406.9 | 276 |
| Glass temperature Tg [°C] | 19.6 | 20.2 |
| Intrinsic viscosity nₘₕ [dl/g] in CHCl₃ * | 1.28 | 1.41 |

| | | |
|---|---|---|
| *at 20°C and a concentration of 10 mg/ml | | |

### EXAMPLE 2: Preparation of PEC nanoparticles by using the solvent displacement method

The PEC-nanoparticle suspension is prepared according to the following modified solvent displacement method:
First of all, a polymer-acetonitrile-stock solution of PEC 99 and PEC 95 is created. Acetonitrile is used, as it is miscible in water and PEC dissolves therein. 100 mg PEC is weighed in a purple cap and is dissolved within approximately 24 hours in 10 ml acetonitrile. The stock solution has a concentration of 10 mg polymer/ml.

The preparation of the stabilizing solution uses 100 mg Mowiol 18/88 under the addition of 200 ml ultra pure water (end concentration: 0.05% PVA). The solution is heated for 3 hours at approximately 90° on a magnetic stirrer in order to dissolve the PVA completely. The solution is filtrated after cooling down through a 0.2 µm cellulose acetate filter and is stored in a refrigerator.

For preparing the nanosuspensions, 5 ml of the PVA-solution (room temperature) is added into a glass vial cleaned by pressed air and the solution height and the charge is marked on the outside.

For the intended end concentration, the desired amount of polymer is taken from the PEC/acetanitrile-solution with a pipette and is added to a 1.5 ml Eppendorf cup. Afterwards, acetonitrile is added in order to obtain an end volume of 1.5 ml. The cup is closed and homogenized with a vortexer. The obtained solution is aspirated in a 2 ml syringe (B Braun) with a 23 Gauche-canula and the air is pressed out, in order to minimize turbulences during injection. Afterwards, the canula is dipped in the middle of the PVA-solution and the piston is quickly and constantly pressed down.

The vials are afterwards evaporated over night under an extractor hood without the use of a magnetic stir bar. The elimination of the magnetic stir bar prevents the aggregation of the PEC. After evaporation, it is checked that no acetonitrile residues remain, and the evaporated water is added up to the marking.

### EXAMPLE 3: Preparation of loaded PEC nanoparticles

For the cell assays, nanoparticle suspensions are produced which are loaded with the fluorescence marker coumarin-6 (Sigma Aldrich), which is used as a model drug. Structure of coumarin-6 (C-6): 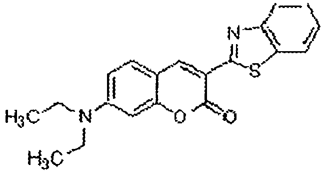 Coumarin - 6 is used (absorption: 485 nm, emission: 505-525 nm) as it allows to optically detect a potential phagocytosis process and furthermore, is a suitable model for a lipophilic agent. Due to its lipophilic characteristics and low water solubility, coumarin-6 can suitably be processed by the solvent displacement method, in order to achieve a high packaging rate. The encapsulation efficiency is very high and lies close to 100%. The preparation of the loaded nanoparticles is also performed using the modified solvent-displacement method as described in example 2. Coumarin-6 is used in a stock solution of 50µg/ml in acteonitrile (the stock solution is stored in the refrigerator and protected by light e.g. by using aluminium foil) and the PEC 95 and 99 polymers as a 10mg/ml stock solution in acetonitrile. All samples are set up in triplicate. For loading the nanoparticles, the required amounts of PEC stock solution and coumarin-6 stock solution are mixed, filled up with acetonitrile up to 1.5 ml and vortexed (see table 2). This solution is then injected into the PVA solution according to the usual schema (see above).

In the present experiment, a concentration of 0.2% of coumarin-6 is chosen (related to the mass of the PEC). As the nanoparticles of PEC 95 are smaller than the nanoparticles of PEC 99 at a concentration of 3mg/5ml, a 1% PVA solution is produced for the PEC 95 3 mg /5 ml in triplicate (0.2% coumarin-6) instead of a 0.05% PVA solution.

**Table 2: Scheme for the reaction batches for Coumarin-6 loaded nanoparticles**

| **PEC95/99 concentration (mg/5ml)** | **PEC95/99 stock solution (10mg/ml)** | **Coumarin-6 stock solution (50µg/ml)** | **Acteonitrile** | **Total volume** |
|---|---|---|---|---|
| 0.1 mg | 10 µl | 4 µl | 1486 µl | 1500 µl |
| 3 mg | 300 µl | 120 µl | 1080 µl | 1500 µl |

The determination of the size is performed by the photon correlation spectroscopy with a zetasizer of Malvem instruments.

TEM analysis reveals that coumarin-6 is incorporated into the nanoparticles. The loaded nanoparticles have a round shape with a smooth surface. No aggregates are found.

### EXAMPLE 4: Regulation of nanoparticles size by the concentration of the polymer solution

The preparation of the nanoparticle suspension is performed using a modified solvent-displacement method as described above in Example 2 utilizing three different PEC 95 or PEC 99 concentrations (see table 3). The necessary amounts of the stock solutions are transferred with a pipette into an Eppendorf cup and are filled up with acetonitrile to 1.5 ml. After vortexing and injection into the PVA solution the desired end concentrations are obtained. All other reaction conditions (reaction volume, temperature, injection conditions, evaporation time and temperature) remain constant. The reactions are prepared in triplicate, and the whole experiment is repeated at least once.

**Table 3: Scheme for the reaction batches for unloaded nanoparticles**

| **PEC concentrations (mg/5ml)** | **PEC stock solution (10mg/ml)** | **Acteonitrile** |
|---|---|---|
| 0.1 mg | 10µl | 1490 µl |
| 3 mg | 300 µl | 1200 µl |
| 6 mg | 600 µl | 900 µl |

The particle size of the obtained nanoparticles is determined by photon correlation spectroscopy (PCS) using a "Zetasizer Nano ZS" (Malvem Instruments).

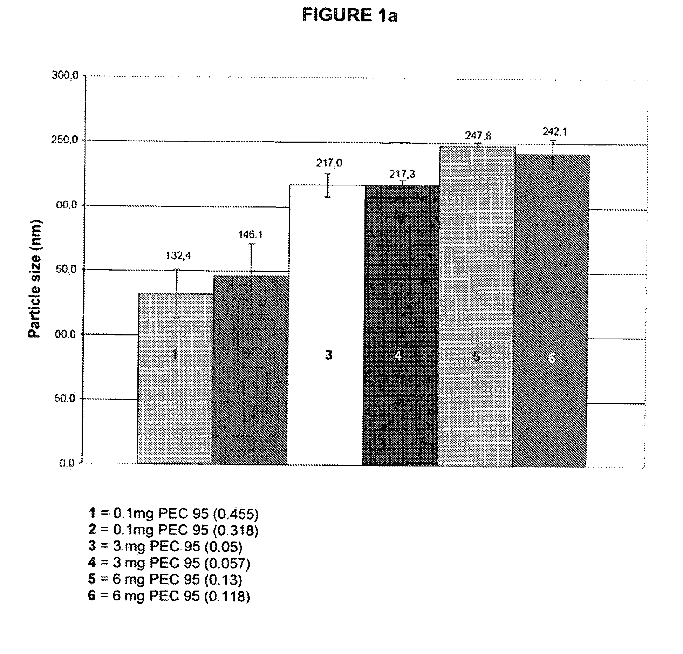

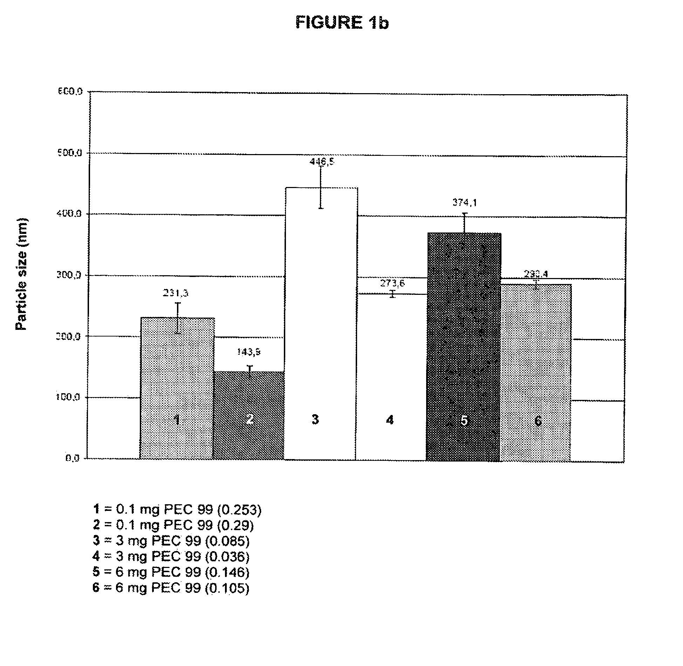

The results of the PCS as shown in Figure 1 demonstrate increased particle size with increasing polymer concentrations. Thus, the size of the nanoparticles may be regulated by the polymer concentration. For PEC 95 (see Figure 1a), more uniform size distributions are obtained than for PEC 99 (see Figure 1b). Best results are obtained with a concentration of 3 mg polymer per 5ml as the polydispersion index is rather narrow. Polymer concentrations of 0.1 mg / 5 ml are very suitable to produce nanoparticles having a diameter of less than 200 nm. Concentrations of 3 mg /5 ml usually lead to nanoparticles having a larger diameter, e.g. above 200 nm.

### EXAMPLE 5: Characterisation of the PEC nanoparticles

### 1. Size determination of PEC nanoparticles loaded with Coumarin-6

Loaded nanoparticle suspensions of PEC 95 and PEC99 are prepared as described above in concentrations of 0.1 mg and 3 mg polymer per 5 ml. The loading is performed with 0.2% (w/w) coumarin-6. When preparing unloaded suspensions of PEC 99, particles having a size of 300 nm and larger are obtained, while PEC 95 delivers more uniform sizes. As nanoparticles having a size of more than 300nm are more interesting for the cell experiments, the size of the PEC 95 nanoparticles can be enlarged with a 1% PVA solution for the concentration 3 mg / 5 ml PVA solution. The PEC 95 (0.1mg/5ml) and PEC 99 (3 mg / 5ml) nanoparticles are prepared as described above. The size is determined in the PVA solution of 0.05% PVA by photon correlation spectroscopy (PCS) using a Zetasizer Nano ZS (Malvern Instrument). The sample of the 3 mg PEC 95 nanoparticules that is prepared in a 1% PVA solution is diluted to 0.05% PVA by using ultrapure water.

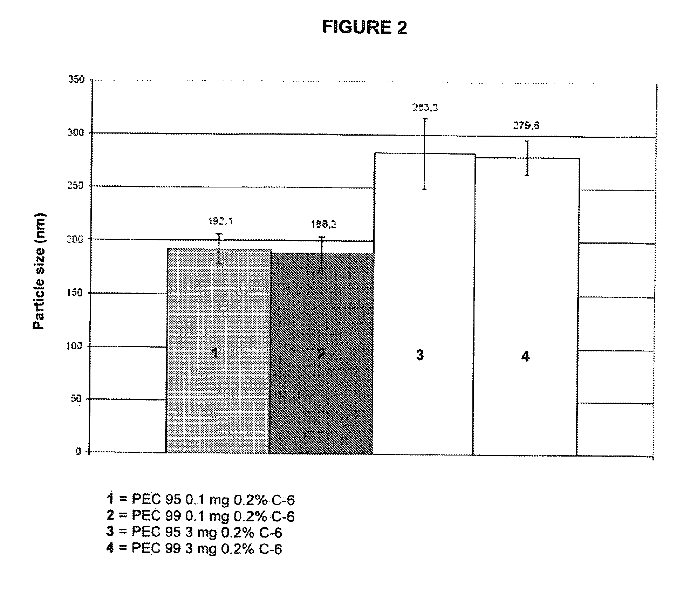

The results of the PCS are shown in Figure 2. In sum the size of the particles is nearly identical to the size of the unloaded particles, as the size is only slightly enlarged. The use of 1% PVA also lead to an increase of size, however not beyond 300 nm as determined by PCS.

### 2. Stability analysis for the PEC 95 and PEC 99 nanoparticles at different temperatures

For the climate experiments, triplicates of the nanoparticle suspension of PEC 95 and PEC 99 are prepared at a concentration of 3mg/5ml at different temperatures.

The nanoparticles suspensions are prepared at 4°C. Therefore, glass vials filled with the PVA solution are positioned for 2h in a climate incubator in order to adapt to the surrounding temperature. Afterwards, the injection of the PEC/acetonitrile solutions is performed (day 0). After the evaporation process for 48 hours (due to the low temperature) the samples are extracted and the size and the zeta potential are determined by a zetasizer and the vials are re-closed. The vials are kept closed and are stored at 4°C and 3 days after injection a further measurement is performed. The nanoparticle triplicates are kept in parallel in the refrigerator and are subjected to the temperature changes. Each new sample is prepared at a higher temperature and is analysed. 24 hours after the last measurement (day 4) the temperature of the climate incubator is elevated to 14°C and two new triplicates of both PEC polymers are prepared in the same concentration in the climate incubator and accordingly at 14°C. After 24h, the acetonitrile of the new samples is evaporated and the samples are measured by a zetasizer (day 5). The temperature of the climate incubator is elevated to 24°C and after reaching this temperature (day 6) further triplicates of PEC 95 and 99 are prepared. 24h after the preparation of the nanoparticles at 24°C (day 7) a further analysis of the new and the older nanoparticle suspensions is performed and the temperature of the climate incubator is increased to 31°C. After this temperature is reached, two new triplicates are prepared and 24h later (day 10) the triplicates that were prepared at 4°C and 31°C are analysed/measured.

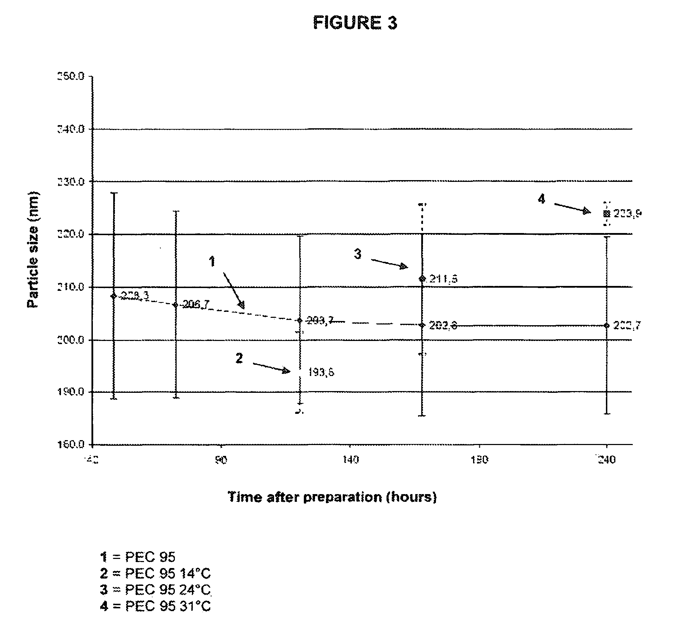

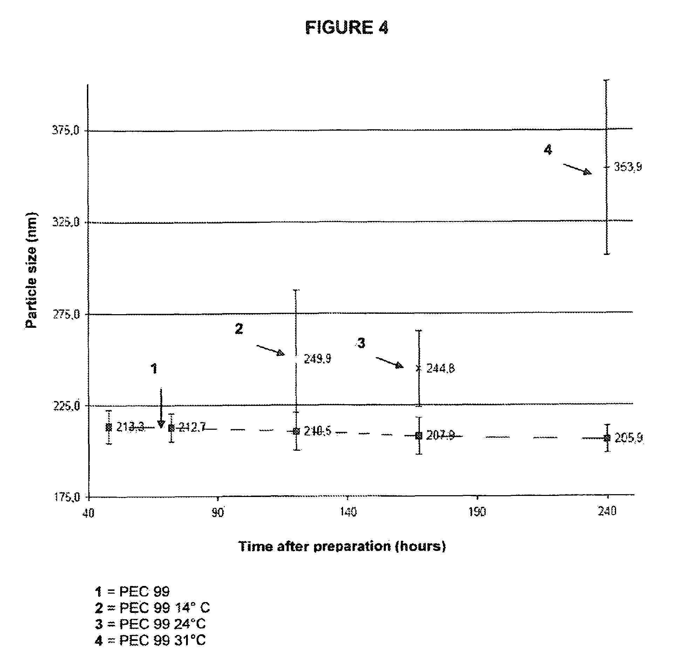

The results for the PEC 95 and PEC 99 nanoparticles are given in Figs. 3 and 4, respectively. The nanoparticles that were prepared at 4°C show no apparent change in particle size and remain stable over at least 10 days. For the nanoparticles prepared at 14°C, 24°C and 31°C it could be shown that only for a reaction temperature of 31 °C the particle size was significantly increased.

The nanoparticles suspensions prepared from the PEC 95 polymer show that the temperature has little influence on the size of the particles and the zeta potential. The triplicate prepared at 4°C show over the whole temperature range (and thus also over the glass transition temperature) basically the same size and zeta potential. For the triplicates that are prepared at temperatures higher than the glass transition temperature, a small increase in size was detected. The temperature accordingly has if at all only a little influence during the preparation of the particles, however, not during storage of the unloaded suspensions.

### 3. Size and swelling analysis for unloaded nanoparticles

From the PEC 95 and PEC 99 nanoparticle reaction mixture aliquots are taken shortly after injection of the polymer solution into the PVA solution (T=0), during the evaporation at t= 30 min, 60 min, 90 min, 120 min. 3h, 4h, 5h, 6h, 7h and 24h. Thereafter, the evaporation process is completed and the vials are sealed with a lid. Further measurements are performed from the final sealed nanoparticle container at t= 48h, 4d, and 9 days after T0.

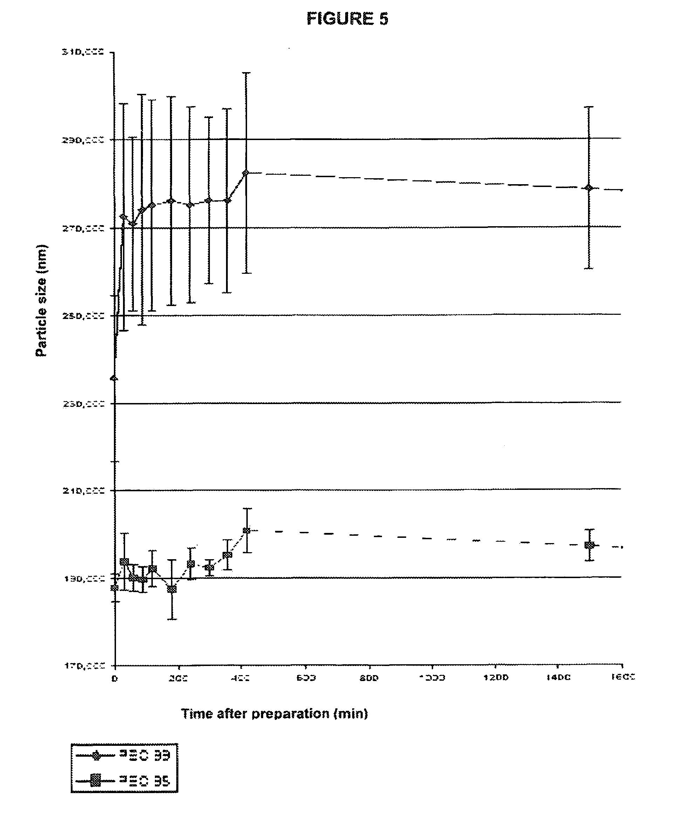

The aliquots are analysed for particle size by PCS as described above. The results for the PEC95 and PEC99 nanoparticles for the first 24 hours are given in Figure 5. For both polymers an increase in particle size during the first 30 min could be observed. After t=30 min the particle size remained constant for the residual examination period:

**Table 4: Results for PEC 99 and PEC 95**

| **Sample** | **Time after T0/min** | **Size/nm** |
|---|---|---|
| PEC 99 | 0 | 235,7 |
| | 30 | 272,5 |
| | 60 | 270,9 |
| | 90 | 274.1 |
| | 120 | 275,2 |
| | 12960 | 267,3 |
| | | |

| **Sample** | **Time after T0/min** | **Size/nm** |
|---|---|---|
| PEC 95 | 0 | 187,9 |
| | 30 | 193,8 |
| | 60 | 190,1 |
| | 90 | 189,7 |
| | 120 | 192,2 |
| | 12960 | 189,3 |

Based on this data it can be concluded that the nanoparticles are basically matured within 30 minutes and do not undergo further major changes during the evaporation period and up to 9 days of storage. The parallel analysis of the nanoparticles by atomic force microscopy reveals that the increase of particle size during the first 30 min is indeed caused by swelling of single particles and not by coalescence.

A further experiment using AFM is performed in order to clarify whether the increase in size is due to the swelling of the particles or is due to a coalescence and aggregation of the articles. For this purpose the nanoparticles are analysed by atomic force microscopy (AFM) using a "JEM 3010" (Jeol GmbH). The experiments are performed in order to determine the size, particle distribution and surface characteristics and to analyse the swelling characteristics.

The nanoparticules with PEC concentrations (PEC 95 and PEC 99) of 0.1 mg / 5 ml and 3 mg / 5 ml are prepared and measured by PCS on the following day. Afterwards, the analysis of the particle distribution and the surface characteristics is performed using AFM (see below) for the first part of the analysis to determine the surface characteristics and the particle distribution.

For the swelling analysis, the nanoparticles development and the swelling characteristics is determined by AFM as described above. The first sample drawing for AFM analysis is performed at T0, and thus directly after injection of the acetonitrile/PEC solution into the 0.05% PVA solution.

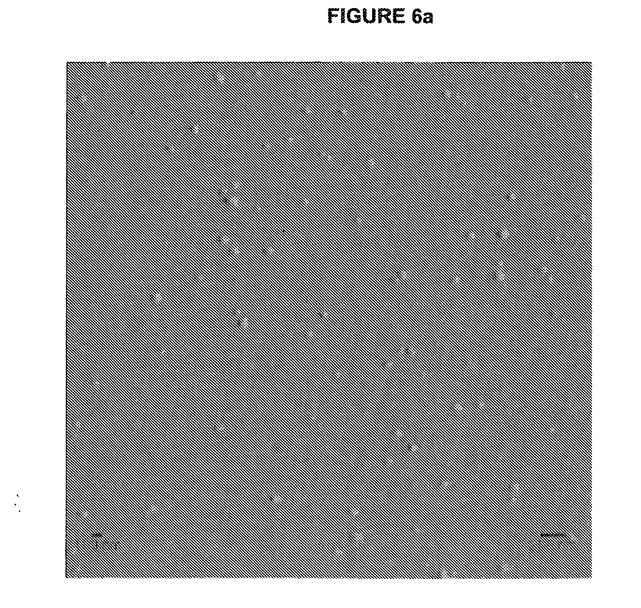

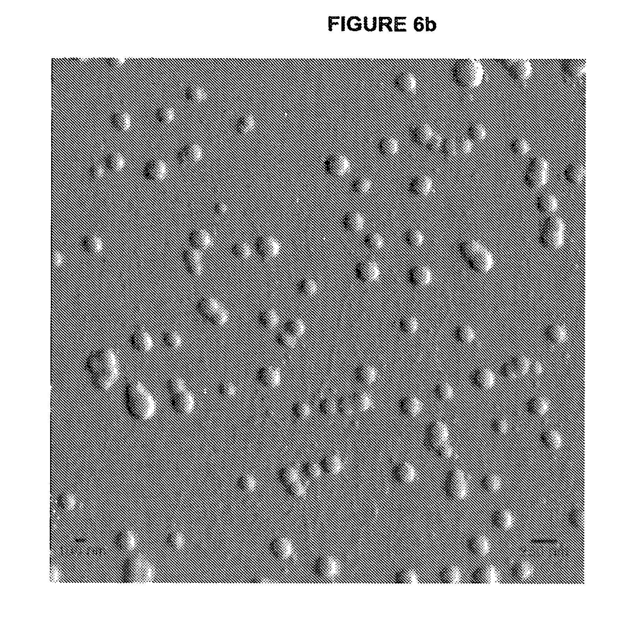

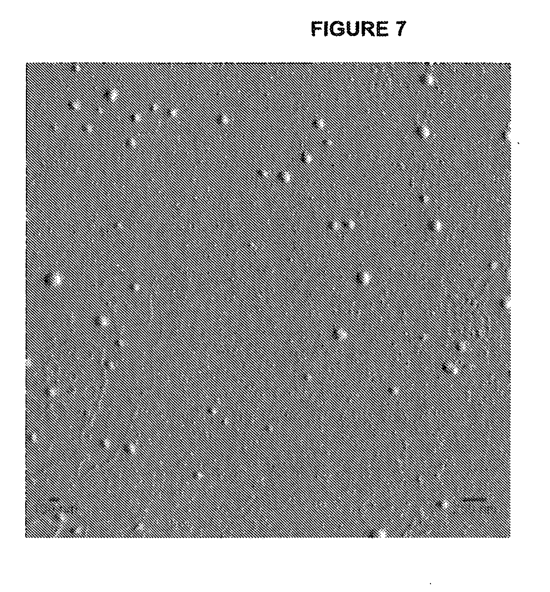

For AFM, a few drops of the nanoparticle suspension are applied on a glass slide. After adsorption of the nanoparticles to the surface the overlying liquid is removed by tapping and the glass slide is dried. After drying, the glass slide is entered into the AFM. In order to preserve the particles the microscopic analysis is performed with a Si₃N₄ tip in tapping mode at 160 kHz. The analysis is performed using the "JPK"-software and the subsequent size measurement using the "ImageJ"-software.
Exemplary results are shown in Figures 6a, 6b and 7.

### PEC 95 (0. 1 mg / 5 ml)

The PCS measurement indicates a size of 200.5 nm with a PDI of 0.295. The AFM results are shown as Figure 6a. The large dots correspond to the nanoparticles. The AFM measurement shows particles having a round shape with a smooth surface. Apparently, the diameter measured by PCS does not correspond to the true size, as the AFM shots indicate a smaller size. The particles have an average size of 100nm (standard deviation 28nm) according to AFM measurement. However, important facts that can be deducted therefrom are the confirmation of the smooth, round shape and the fact that the particles are present as singular particles and not as aggregates.

### PEC 95 (3 mg / 5 ml)

For the PEC 95 (3 mg / 5 ml) nanoparticles the analysis showed an average diameter of 202nm which is less than the 235.9 nm (with a PDI of 0.03) observed by PCS. However the particle size measured by PCS is presumed to be larger since FCS detects the particle including its solvent layer. The photos showed round particles with a smooth surface which are nearly all deposited as individual particles without signs of coalescence. The results are shown in Figure 6b.

### The PEC 99 (0.1 mg / 5 ml)

The particles were similar in appearance. The diameter was determined as 106 nm in comparison with 139.8 nm measured by PCS (with a PDI of 0.216). The PEC 99 nanoparticles (3 mg /5 ml) had a stronger tendency to form aggregates and are larger.

The results indicate that measurements by AFM are more accurate to determine the size of the nanoparticles.

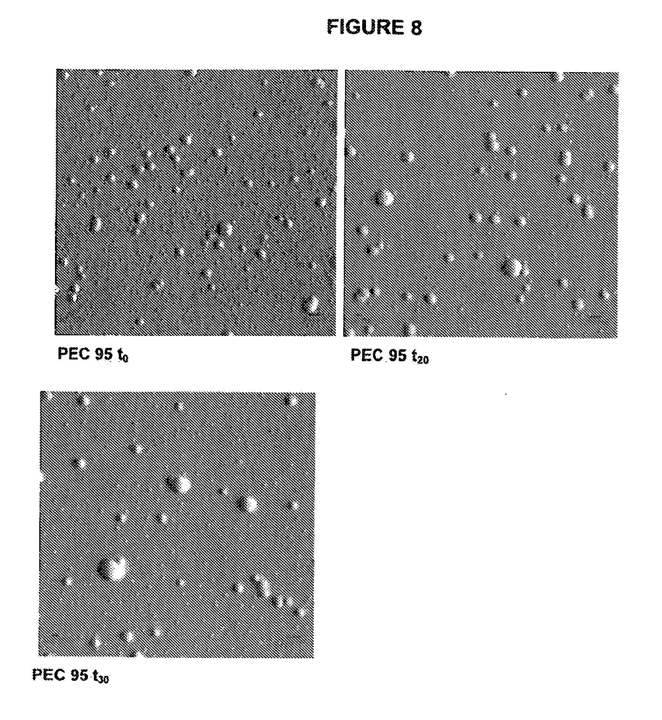

The results of the swelling experiments with the respective unloaded PEC nanoparticles are shown in Figure 8 (for PEC 95 (3 mg / 5 ml). The results show an increase in the size of the PEC 95 nanoparticles within the first half hour of the evaporation process. As is indicated by the graphs, most of the particles do not coalesces but increase their size due to a swelling process. The nanoparticles of PEC 99 showed a larger increase in size than the PEC 95 particles within the first hour of the evaporation process and were more susceptible to coalescence.

**Table 5: Results for PEC 95 and PEC 99**

| **Polymer** | **Time/min** | **Size/nm** | **Standard deviation/nm** |
|---|---|---|---|
| PEC 95 | 0 | 154 | 46 |
| PEC 95 | 20 | 227 | 81 |
| PEC 95 | 30 | 241 | 118 |
| PEC 99 | 0 | 261 | 49 |
| PEC 99 | 20 | 450 | 118 |
| PEC 99 | 30 | 400 | 91 |

### EXAMPLE 6: Uptake of coumarin-6 into macrophages cultured in vitro

In order to analyse the uptake of the coumarin-6 loaded nanoparticles, the murine macrophage cell line J744A.1 (DSMZ Braunschweig) is cultured at 37°C and 8.5% CO₂ with a relative humidity of 95%. The cells are fed every two days with DMEM (PAA ready to use medium), glucose, glutamine and 10% fetal calf serum (FCS Cytogen). After reaching the critical cell number, the culture is splitted (usually 2 to 3 times per week). For the analysis under CSLM (confocal laser scanning microscopy) the nuclei of the cells are stained with DAPI: 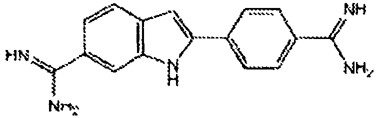 In order to quench the fluorescence of the non-phagocytised, Fluorescing nanoparticles, the non fluorescent dye trypan blue is added to the medium: 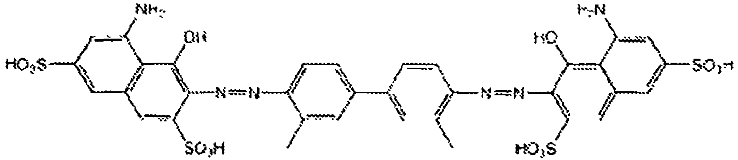 Since this dye is not incorporated by living cells, only the fluorescence outside the cells is quenched.

The cells are analysed by CLSM in order to analyse how the loaded nanoparticles are processed by the cells. The confocal microscopy is performed using an Axiovert 100M and 510 Scanning device (Zeiss) and analysed using the LSM Image Browser software. The CSLM analysis only allows a qualitative analysis.

By making optical cuts along the Z-axis one can determine whether a fluorescence of coumarin-6 occurs inside or outside the cell. As a control, a part of the macrophages is incubated at 4°C. At this temperature, energetic processes of the cell are inhibited and accordingly also the phagocytosis process.

The PBS buffer used herein has the following concentration: KCl 0.2 g; NaCl 8.0 g; KH₂PO₄ 0.2 g: Na₂HPO₄ 1.51 g, aqua dest. ad 1000 ml). For the cell experiments, a 10X concentrated PBS buffer is necessary. For this purpose, the buffer is filled up with aqua dest. to 100 ml instead of 1000 ml. The buffer is adjusted to pH 7 and sterile filtered using a 0,2µm filter.

For the first part of this experiment, different charges of PEC 99 nanoparticles in concentrations of 0,1 mg and 3mg polymer per 5ml with 0.2% coumarin-6 are prepared using the known schema (see above). For the experiment a 0.1mg/5ml charge with 126nm and a 3mg/5ml charge with 297nm sized particles are chosen.

For the experiment, J774-cells with a density of 0.5 x 10⁴/well are seeded out in two chamber slides with a working volume of 400µl 48 hours before the uptake experiment. The present medium is aspirated and 320µl PEC 99 0.1mg nanoparticles suspension, 40µl 10x concentrated PBS buffer and 40µl mouse serum is added (end concentration of PEC: 16µg/ml). In six further chambers 320µl PEC 99 3mg nanoparticles suspension was added, again under addition of 40µl mouse serum and 10x PBS buffer (end concentration of PEC: 480µg/ml). 4 chambers of the slides remain with medium and without the addition of nanoparticles in order to have comparative probes of untreated cells. The cells are incubated for 1 hour at 37°C. Afterwards the medium is aspirated and the cells are washed twice with PBS und are stained using DAPI. The staining is performed for all cells incubated with nanoparticles and for two chambers of the comparative cells. The cells of two chambers are only fixed but not stained in order to analyse whether DAPI emits fluorescence in area of coumarin-6. The chambers are removed from the slides at the end of the experiment and are covered with a cover slide using few drops of Fluorsave (Clabiochem). The samples are stored protected from light and shortly afterwards analysed by CLSM.

The second part of the experiment analyses the uptake of the nanoparticle suspension in different concentrations at 37°C and 4°C. For this experiment, a new charge PEC 99 3mg with 0.2% coumarin-6 is prepared (diameter: approximately 300nm).

48 hours before the beginning of the experiment the macrophages are seeded in three chamber slides at a density of 0.5 x 10⁴/well. For the incubation of the cells at 4°C a chamber slide is stored in a refrigerator. For the analysis of concentration-dependency, concentrations of the PEC suspension of 480µg/ml, 320µg/ml, 120µg/ml (referred to the end concentration in the chambers of the slides) are chosen. Therefore, the medium is aspirated in all but four chambers (comparative samples). 10% mouse serum and PBS buffer is added and the nanoparticles suspension is added depending on the desired end concentration (80µl for 120µg/ml, 160µl for 240µg/ml or 320µl for 480µg/ml). 4 chambers are incubated with the PEC nanoparticles at the different concentrations at 37°C for an hour. The incubation for more than an hour at 4°C is performed in a pre-cooled slide in 4 chambers with 480µg/ml and 240 µg / ml PEC nanoparticles. As negative control, 4 chambers of the cells stored at 37°C are not incubated with nanoparticles.

After incubation of one hour, the liquid is aspirated, the cells are washed twice with PBS and fixed and stained with DAPI (see Example 7). Afterwards, the chambers are removed from the slides and covered with a cover slide using few drops of Fluorsave (Clabiochem). The samples are stored protected from light and shortly afterwards analysed by CLSM.

The results show for the first part of the analysis (qualitative analysis) that the incubation with the PEC nanoparticles (126nm and 297nm) led to a fluorescence within the cells. In order to analyse whether the uptake occurred via active phagocytosis, the second part of the experiment is performed at the different temperatures. If the PEC nanoparticles are processed by an active phagocytosis process, the cells incubated at 4°C should show no or a considerably decreased fluorescence. The CSLM results showed an increase of the fluorescence corresponding to the increase in the nanoparticle concentration. However, also the cells incubated at 4°C with 480µg/ml and 240µg ml nanoparticles showed a fluorescence. This indicates that the PEC nanoparticles lower active phagocytosis by the macrophages.

### EXAMPLE 7: DAPI-staining (for a 15 ml working volume)

2 ml of a fixation solution (one part PBS and one part 4 % paraformaldehyde) is added to the cells with medium and afterwards, the complete liquid is aspirated. Afterwards, two times 5 ml fixation solution is added to the cells, incubated for 5 minutes and aspirated. Afterwards, the cells are dried for 15 min at the air and incubated with the DAPI solution (1 ml PBS + 20 µl DAPI) for 30 minutes in the dark. At the end of the staining process the DAPI is aspirated and washed three times with PBS.

### EXAMPLE 8: Comparison of the phagocytosis of PEC 99 and PEC 95 nanoparticles with fluorescent polystyrene latex beads as standard via FACS at 37 and 4° and upon addition of NaN₃

Fluorescence activated cell sorting (FACS) provides the possibility to analyze and characterise a large number of cells regarding their size, compactness and fluorescence within a short time period.

The aim of this experiment is to determine by FACS, whether the PEC nanoparticles of different sizes are better or worse uptaken/processed by macrophages than fluorescently labelled polystyrol standards (PSS), which are activated at 468 nm and emit fluorescence at 508 nm. One control is performed by adding NaN₃ as an inhibitor of phagocytosis. The other control is performed at an incubation temperature of 4° C as the cell processes and accordingly the phagocytosis is inhibited/slowed down at this temperature.

| **Material** | **Manufacturer** |
|---|---|
| NaN₃ | Acros |
| DAPI | Invitrogen Karlsruhe |
| FACS Scan | BD Biosciences, San |
| FACS Flow | BD Biosciences, San |
| FCS Express Software | Devonsoftware |
| Fluorescing polystyrol standard 100nm, | Duke scientific corporation |
| Fluorescing polystyrol standard 500nm | Duke scientific corporation |
| Trypan blue | Invitrogen |

For the first experiment, J774 cells are seeded out at a density of 6 x 10⁴/Well in a 24 well plate (working volume 1 ml) 24 hours before the beginning. In order to determine the phagocytosis rate by the macrophages, nanoparticle suspensions of PEC 95 and PEC 99 at a concentration of 0,1 mg and 3 mg and 0.2 % coumarin-6 per 5 ml are produced. Additionally, larger nanoparticles of the type PEC 95 are prepared, by using a 1 % PVA solution as stabilizer. As a negative control, 6 wells of the plate are left untreated with the macrophages. For the incubation with the nanoparticle suspensions, the medium of the remaining wells is aspirated and PEC 95-NP 0.1 mg (137 nm) and 3 mg (239 nm) and PEC 99-NP 0.1 mg (133 nm) and 3 mg (283 nm) are added in triplicate under addition of 10 % mouse serum and PBS. The end concentration in the wells is for each triplicate of the 0.1 mg and 3 mg PEC-NP 16 µg/ml and additionally one triplicate with 3 mg PEC-NP with 480 µg/ml. The incubation is performed at 37° for one hour in an incubator. After the end of the incubation term, the suspensions and the medium in case of the control cells is aspirated and washed two times with PBS. In order to quench fluorescent nanoparticles at the surface of the cells, the cells are incubated with 0.4 % trypan blue for 5 min and washed twice with PBS. Afterwards, 100 µl trypsin is added and the well plates are pivoted in order to remove the macrophages from the well plate. 200 µl paraformaldehyde: FACS flow (1:1) is added to the cells and the samples are transferred to a glass pipe and analysed via FACS.

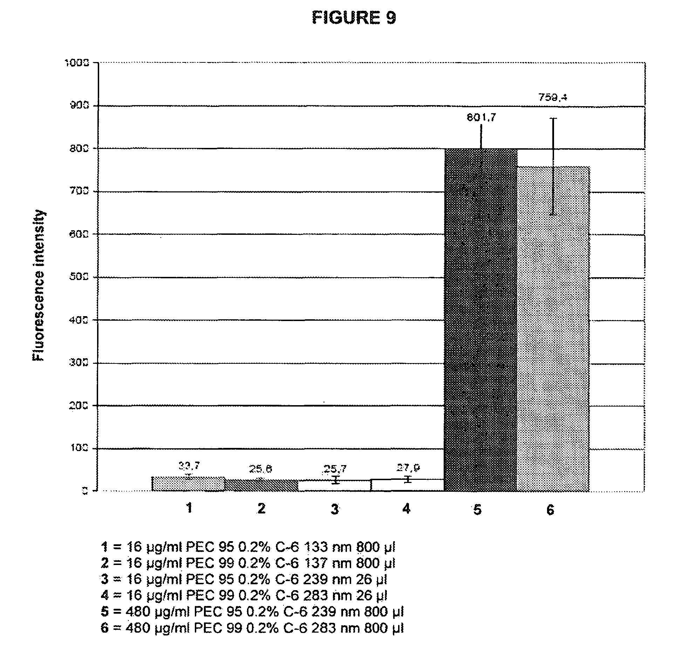

The aim of this first experiment is to analyse whether there are differences in the phagocytosis of loaded PEC 99 and PEC 95 nanoparticles at the same concentration. The results are shown in Figure 9. The indicated fluorescence values correspond to the arithmetic mean value of the fluorescence determination after the subtraction of the fluorescence of the blind probe, namely the untreated cells. The results show that the fluorescence within the cells depends predominantly on the used nanoparticle concentration.

The aim of the second experiment is to analyze per FACS the fluorescence of the macrophages after incubation of PEC 99 and PEC 95 compared to the fluorescence after the addition of fluorescent polystyrol nanoparticles, which are standard polymeric nanoparticles. The experiments are performed at 4° and 37°. In order to use a further possibility to inhibit the active phagocytosis process, some cells of the 37° experiment are incubated with the phagocytosis inhibitor NaN₃. Therefore, a 60 mM NaN₃ solution is prepared, e.g. by dissolving 39 mg NaN₃ in 10 ml ultra pure water.

The cells are seeded out 24 hours before the beginning of the experiment at a density of 6 x 10⁴/well in two 24 well plates. Afterwards, nanoparticle suspensions of PEC 95 and PEC 99 are prepared which are loaded with 0.2 % coumarin-6 in the concentrations of 0.1 mg and 3 mg per 5 ml.

One hour before the beginning of the experiment one well plate is deposited at 4° in a refrigerator. 3 hours before the beginning of the experiment, half of the cells of the other plate that is stored at 37°C. are supplemented with 500 µl NaN₃ and 500 µl medium after aspiration of the medium (end concentration NaN₃: 30 mMol/l). After 3 hours of incubation in the NaN₃ solution, respectively one hour storage at 4° the cell experiments are started. Therefore, the media in the wells are aspirated (except for the blind samples). To each well of the plate that is cooled at 4°, a suspension of the PEC-NP 0.1 mg (108 nm), PEC-95 3 mg (285 nm), PEC-99 0.1 mg (110 nm) and PEC-99 3 mg (297 nm) is added. The end concentration of the PEC nanoparticles in the wells after the addition of 10 % mouse serum and PBS buffer for all samples amounts to 16 µg/ml. Furthermore, the fluorescent polystyrol standard of the sizes 100 nm and 500 nm are added to separate wells under the addition of 10 % mouse serum and PBS. The end concentration of the standards in the wells is 20 µg/ml.

The cells that were stored at 37°C are equally prepared with particles, respectively on the side (half) treated with NaN₃ and the side (half) that was not non-treated with NaN₃ respectively.

After the addition of the particles the cells are incubated for one hour at 4°C or 37°C and afterwards the suspensions are aspirated, washed twice with PBS and after addition of 0.4 % trypan blue incubated for 5 min. Afterwards, the cells are again washed with PBS, trypsinized and transferred with FACS flow: paraformaldehyde (see above) to a glass tube.

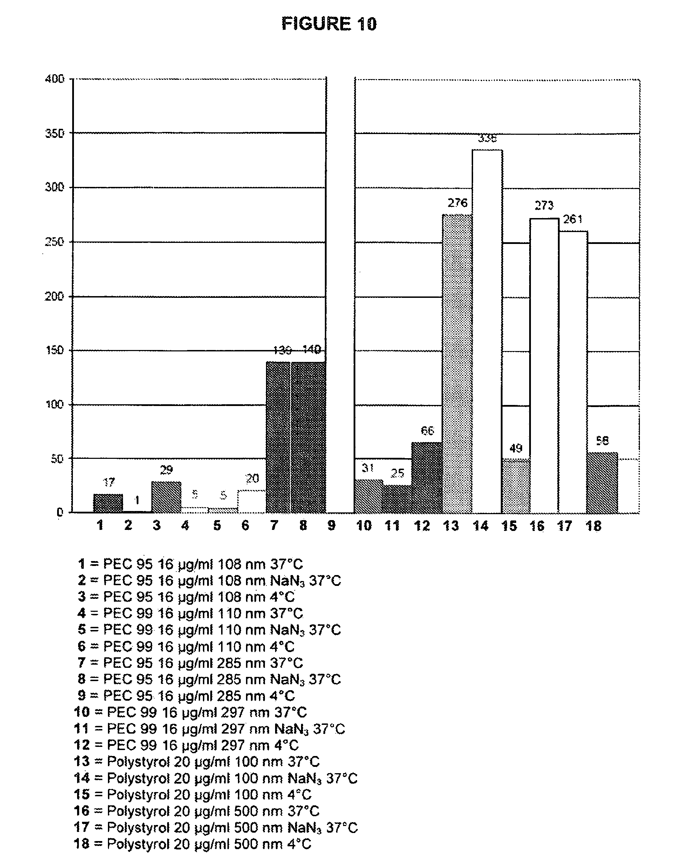

The results of the FACS analysis are shown in Figure 10. The indicated fluorescence values correspond to the arithmetic mean value of the fluorescence determination after the subtraction of the fluorescence of the blind probe, namely the untreated cells. The intensity of the fluorescence is a measure for the extent of phagocytosis of the nanoparticles. The polystyrol standards show the expected high levels of fluorescence under the conditions where phagocytosis occurs (37°C, no inhibitor) and a low fluorescence in case the phagocytosis is inhibited/slowed down (4°C or addition of NaN₃), The polystyrene nanoparticles are thus phagocytised efficiently which makes them biologically unstable as they are eliminated quickly by the macrophages in the body. The results for the PEC nanoparticles of the present invention show a remarkably different profile, as there is no significant difference when the phagocytosis is inhibited/slowed down (see results at 4°C and with NaN₃).

Especially for the smaller nanoparticles the difference in phagocytosis between polystyrene-NP and PEC-NP is large as can be derived when comparing the low fluorescence values (= low level of phagocytosis) of PEC 95 16 µg/ml 108nm 37°C and °PEC 99 16 µg/ml 110nm 37°C" versus the high fluorescence value (= high level of phagocytosis) for "polystyrol 20µg/ml 100 nm 37°C". Thus, PEC nanoparticles according to the present invention are much less attacked by macrophages, thereby allowing using the PEC nanoparticles as parenteral depot which is releasing an encapsulated pharmacologically active agent upon biodegradation and making the pharmacologically active agent systemically available over the entire, respectively longer release period. Furthermore, the results show that smaller PEC nanoparticles are even less phagocytised than larger PEC nanoparticles.

## Claims

1. A pharmaceutical composition comprising nanoparticles comprised of at least one pharmacologically active agent, at least one poly(ethylene carbonate) (PEC) polymer, the pharmaceutical composition being in the form of a nanoparticle suspension.

2. The pharmaceutical composition according to claim 1, which is a parenteral formulation.

3. The pharmaceutical composition, according to claim 1 or claim 2, wherein the nanoparticle have a size of less than 1000 nm.

4. The pharmaceutical composition, according to one of the claims 1 to 2, wherein the nanoparticles have a size of less than 500 nm.

5. The pharmaceutical composition according to one of the claims 1 to 4, wherein the molecular weight of the PEC polymer is less than 2 000 000 g/mol.

6. The pharmaceutical composition according to one of the claims 1 to 4, wherein the molecular weight of the PEC polymer is less than 500 000 g/mol.

7. The pharmaceutical composition according to one of the claims 1 to 6, wherein the pharmacologically active agent is selected from the group consisting of chemical compounds, biologically active agents, nucleic acids, peptides and proteins.

8. The pharmaceutical composition according to claim 7, comprising a pharmacologically active agent further selected from the group consisting of the class of somatostatin analogue inhibitors, biphosphonates, lipid altering drugs, and immunosuppressant agents.

9. The pharmaceutical composition according to claim 1, wherein the PEC has at least one of the following characteristics:
(a) an ethylene carbonate content of 70 to 100 Mol %,
(b) an intrinsic viscosity of 0.4 to 4.0 dl/g as measured in chloroform at 20 °C, and/or
(c) a glass transition temperature of from 5 to 50 °C

10. The pharmaceutical composition according to claim 1, wherein the PEC nanoparticle suspension has sustained release characteristics.

11. The pharmaceutical composition according to claim 1, wherein the PEC nanoparticle suspension is a parenteral depot formulation.

12. The pharmaceutical composition according to claim 1, wherein the nanoparticle suspension further comprises pharmaceutically acceptable excipients or additives.

13. A method of manufacturing pharmaceutical composition according to at least one of the claims 1 to 12 by preparing a suspension of nanoparticles comprising at least one pharmacologically active agent and at least one poly(ethylene carbonate) polymer.

14. The method according to claim 13, wherein the nanoparticle suspension is prepared using the solvent displacement method, the solvent evaporation method or the salting out method.

15. The method according to claim 13 or 14, wherein the particle size is regulated by the varying the polymer concentration.

## Patentansprüche

1. Pharmazeutische Zusammensetzung, umfassend Nanopartikel, die aus mindestens einem pharmakologisch aktiven Mittel und mindestens einem Poly(ethylencarbonat) (PEC)-Polymer bestehen, wobei die pharmazeutische Zusammensetzung in Form einer Suspension von Nanopartikeln vorliegt.

2. Pharmazeutische Zusammensetzung nach Anspruch 1, die eine parenterale Formulierung ist.

3. Pharmazeutische Zusammensetzung nach Anspruch 1 oder Anspruch 2, wobei die Nanopartikel eine Größe von weniger als 1000 nm aufweisen.

4. Pharmazeutische Zusammensetzung nach einem der Ansprüche 1 bis 2, wobei die Nanopartikel eine Größe von weniger als 500 nm aufweisen.

5. Pharmazeutische Zusammensetzung nach einem der Ansprüche 1 bis 4, wobei das Molekulargewicht des PEC-Polymers weniger als 2 000 000 g/mol beträgt.

6. Pharmazeutische Zusammensetzung nach einem der Ansprüche 1 bis 4, wobei das Molekulargewicht des PEC-Polymers weniger als 500 000 g/mol beträgt.

7. Pharmazeutische Zusammensetzung nach einem der Ansprüche 1 bis 6, wobei das pharmakologisch aktive Mittel aus der Gruppe ausgewählt ist, die aus chemischen Verbindungen, biologisch aktiven Mitteln, Nucleinsäuren, Peptiden und Proteinen besteht.

8. Pharmazeutische Zusammensetzung nach Anspruch 7, die ein pharmakologisch aktives Mittel umfasst, das weiter aus der Gruppe ausgewählt ist, die aus der Klasse der Inhibitoren von Somatostatinanaloga, Bisphosphonaten, Lipid-verändernden Arzneimitteln und immunsupprimierenden Mitteln besteht.

9. Pharmazeutische Zusammensetzung nach Anspruch 1, wobei das PEC mindestens eine der folgenden Eigenschaften besitzt:
(a) einen Ethylencarbonatgehalt von 70 bis 100 Mol-%,
(b) eine intrinsische Viskosität von 0,4 bis 4,0 dl/g gemäß Messung in Chloroform bei 20 °C und/oder
(c) eine Glasübergangstemperatur von 5 bis 50 °C.

10. Pharmazeutische Zusammensetzung nach Anspruch 1, wobei die Suspension von PEC-Nanopartikeln für eine anhaltende Freisetzung sorgende Eigenschaften besitzt.

11. Pharmazeutische Zusammensetzung nach Anspruch 1, wobei die Suspension von PEC-Nanopartikeln eine parenterale Depotformulierung ist.

12. Pharmazeutische Zusammensetzung nach Anspruch 1, wobei die Suspension von Nanopartikeln des Weiteren pharmazeutisch akzeptable Hilfsstoffe oder Additive umfasst.

13. Verfahren zum Herstellen einer pharmazeutischen Zusammensetzung nach mindestens einem der Ansprüche 1 bis 12 durch Herstellen einer Suspension von Nanopartikeln, umfassend mindestens ein pharmakologisch aktives Mittel und mindestens ein Poly(ethylencarbonat)-Polymer.

14. Verfahren nach Anspruch 13, wobei die Suspension von Nanopartikeln unter Verwendung des Lösemittelverdrängungsverfahrens, des Lösemittelabdampfverfahrens oder des Aussalzverfahrens hergestellt wird.

15. Verfahren nach Anspruch 13 oder Anspruch 14, wobei die Partikelgröße durch die Veränderung der Polymerkonzentration gesteuert wird.

## Revendications

1. Composition pharmaceutique comprenant des nanoparticules composées d'au moins un agent pharmacologiquement actif, d'au moins un polymère de carbonate de polyéthylène (PEC), la composition pharmaceutique se présentant soue la forme d'une suspension de nanoparticules.

2. Composition pharmaceutique selon la revendication 1, qui est une formulation parentérale.

3. Composition pharmaceutique selon la revendication 1 ou 2, dans laquelle les nanoparticules ont une taille inférieure à 1 000 nm.

4. Composition pharmaceutique selon l'une des revendications 1 ou 2, dans laquelle les nanoparticules ont une taille inférieure à 500 nm.

5. Composition pharmaceutique selon l'une des revendications 1 à 4, dans laquelle le poids moléculaire du polymère PEC est inférieur à 2 000 000 g/mol.

6. Composition pharmaceutique selon l'une des revendications 1 à 4, dans laquelle le poids moléculaire du polymère PEC est inférieur à 500 000 g/mol.

7. Composition pharmaceutique selon l'une des revendications 1 à 6, dans laquelle l'agent pharmacologiquement actif est sélectionné dans le groupe constitué par les composés chimiques, les agents biologiquement actifs, les acides nucléiques, les peptides et les protéines.

8. Composition pharmaceutique selon la revendication 7, comprenant un agent pharmacologiquement actif sélectionné en outre dans le groupe constitué par la classe des inhibiteurs des analogues de la somatostatine, des biphosphonates, des médicaments modifiant les lipides et des agents immunosuppresseurs.

9. Composition pharmaceutique selon la revendication 1, dans laquelle le PEC a au moins l'une des caractéristiques suivantes :
(a) une teneur en carbonate d'éthylène de 70 à 100 % en moles,
(b) une viscosité intrinsèque de 0,4 à 4,0 dl/g mesurée dans le chloroforme à 20°C, et/ou
(c) une température de transition vitreuse de 5 à 50°C.

10. Composition pharmaceutique selon la revendication 1, dans laquelle la suspension de nanoparticules de PEC a des caractéristiques de libération prolongée.

11. Composition pharmaceutique selon la revendication 1, dans laquelle la suspension de nanoparticules de PEC est une formulation à effet retard pour administration parentérale.

12. Composition pharmaceutique selon la revendication 1, dans laquelle la suspension de nanoparticules comprend en outre des excipients ou des additifs pharmaceutiquement acceptables.

13. Procédé de fabrication d'une composition pharmaceutique selon au moins l'une des revendications 1 à 12 par préparation d'une suspension de nanoparticules comprenant au moins un agent pharmacologiquement actif et au moins un polymère de carbonate de polyéthylène.

14. Procédé selon la revendication 13, dans lequel la suspension de nanoparticules est préparée à l'aide du procédé de déplacement de solvant, du procédé d'évaporation de solvant ou du procédé de relargage.

15. Procédé selon la revendication 13 ou 14, dans lequel la taille des particules est régulée en faisant varier la concentration du polymère.
